(19) Europäisches Patentamt
European Patent Office
Office européen des brevets

(11) Veröffentlichungsnummer: **0 284 828 B1**

# (12) EUROPÄISCHE PATENTSCHRIFT

(45) Veröffentlichungstag der Patentschrift: **16.10.91**

(51) Int. Cl.5: **C08K 5/34**, C07D 233/90, C07D 235/20, C07D 235/24, C08L 75/04

(21) Anmeldenummer: **88103481.3**

(22) Anmeldetag: **05.03.88**

## (54) Gegen Einwirkung von Licht stabilisierte Polymere sowie neue Imidazol-2-carbonsäure-anilide.

(30) Priorität: **14.03.87 DE 3708292**

(43) Veröffentlichungstag der Anmeldung:
**05.10.88 Patentblatt 88/40**

(45) Bekanntmachung des Hinweises auf die Patenterteilung:
**16.10.91 Patentblatt 91/42**

(84) Benannte Vertragsstaaten:
**BE CH DE ES FR GB IT LI**

(56) Entgegenhaltungen:
**US-A- 3 740 413**
**US-A- 4 011 236**

**CHEMICAL ABSTRACTS, Band 101, Nr. 3, 16. Juli 1984, Columbus, Ohio, USA; P.A. PETYUNIN et al. "Synthesis an d properties of benzimidazole-2-car-boxylic acid N-R-amides", S. 594, Spalte 2, Zusammenfassung-Nr. 23 395a**

(73) Patentinhaber: **BASF Aktiengesellschaft**
**Carl-Bosch-Strasse 38**
**W-6700 Ludwigshafen(DE)**

(72) Erfinder: **Spang, Peter, Dr.**
**Auf der Mess 3**
**W-6670 St. Ingbert(DE)**
Erfinder: **Neumann, Peter, Dr.**
**Franz-Schubert-Strasse 1**
**W-6908 Wiesloch(DE)**
Erfinder: **Wagenblast, Gerhard, Dr.**
**Hanns-Fay-Strasse 3**
**W-6710 Frankenthal(DE)**
Erfinder: **Trauth, Hubert**
**Milanstrasse 5**
**W-6724 Dudenhofen(DE)**

**EP 0 284 828 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Aus den US-PS 3 907 700 und 4 011 236 sind N-(Benzimidazol-2-yl)-aryl-carboxamide der Formel (X)

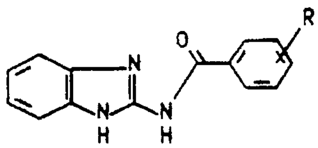

(X)

als UV-Schutzmittel für organische Polymere bekannt. Diese Amide zeigen jedoch z.B. in Polyurethan keine ausreichende Schutzwirkung. Ein weiterer Nachteil ist die Unverträglichkeit mit vielen Kunststoffen, die Flüchtigkeit und die Verfärbung der polymeren Substrate nach dem Einarbeiten der Amide.

Aufgabe der Erfindung war es, Verbindungen bereitzustellen, die eine gute Absorption im Wellenlängenbereich von 280-400 nm, geringe Flüchtigkeit und geringe Tendenz zur Verfärbung bei der thermischen Verarbeitung aufweisen.

Aus den US-PS 3 740 413 und 3 661 925 sind Benzimidazol-2-carbonsäure-anilide als pharmazeutisch wirksame Verbindungen bekannt. Außerdem werden in der FR-PS 1 517 719, den Chem. Ber. 92 (1959), Seite 550, dem J. Chem. Soc. Sect. C 1967, Seite 20, in CA 101, 23 395a und CA 98, 143 322r weitere Benzimidazol-2-carbonsäureanilide und 4,5-Diphenylimidazol-2-carbonsäureanilide beschrieben.

Es wurde gefunden, daß die aromatischen Imidazol-2-carbonsäureanilide der Erfindung diese Eigenschaften besitzen und hervorragend zum Schutz von organischen Materialien gegen die Einwirkung von Licht geeignet sind.

Dementsprechend betrifft die Erfindung ein gegen Einwirkung von Licht stabilisiertes Polymer aus der Gruppe Polyolefine, Polystyrol, Styrolpolymerisate, halogenhaltige Vinylpolymere, Polyacrylate, Polymethacrylate, Polyacrylamide, Polyacrylnitril, Polyvinylalkohol und dessen Acylderivate, Polyacetale, Polyalkylenoxide, Polyphenylenoxide, Polyurethane und Polyharnstoffe, Polysulfone, Polyamide, Polyester, Polycarbonate, vernetzte Polymere aus Aldehyden und Phenolen, Harnstoff und/oder Melamin, ungesättigte Polyesterharze, Alkydharze, duroplastische und thermoplastische Acrylharze, das dadurch gekennzeichnet ist, daß dieses als Lichtschutzmittel in Mengen von 0,01 bis 5 Gewichtsprozent, bezogen auf das zu stabilisierende Polymer, mindestens eine Verbindung der allgemeinen Formel (I)

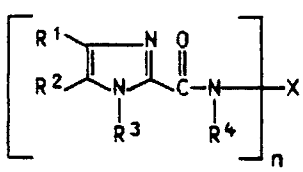

(I)

enthält, in der

n für 1 oder 2,

$R^1$ und $R^2$ oder einer der Reste $R^1$ oder $R^2$ unabhängig voneinander für Phenyl oder Heteroaryl, wobei diese Reste gegebenenfalls durch $C_1$- bis $C_{12}$-Alkyl, $C_1$- bis $C_{12}$-Alkoxy, Phenoxy, Halogen oder Trifluormethyl substituiert sind und der andere der Reste $R^1$ oder $R^2$ für Wasserstoff, $C_1$- bis $C_{12}$-Alkyl, $C_7$- bis $C_9$-Phenalkyl oder für $-COOR^5$, worin $R^5$ lineares oder verzweigtes $C_1$- bis $C_{12}$-Alkyl, $C_5$- bis $C_8$-Cycloalkyl, Phenyl oder $C_7$- bis $C_{10}$-Phenalkyl bedeutet und wobei der Cycloalkylrest und die Phenylreste gegebenenfalls durch $C_1$- bis $C_4$-Alkyl substituiert sind, oder

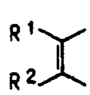

für einen ankondensierten Benzolring, der gegebenenfalls durch Hydroxy, Halogen, $C_1$- bis $C_{12}$-Alkyl, $C_1$- bis $C_{12}$-Alkoxy, $C_7$- bis $C_9$-Phenylalkyl, Phenyl, $-COOR^6$, $-CO-R^6$, $-CO-NH-R^6$, $-COOH$, $-O-COR^6$, $-NH-CO-R^6$, $-SO_2-R^6$ oder -CN substituiert ist, worin $R^6$ lineares oder verzweigtes $C_1$-

bis $C_{12}$-Alkyl, $C_5$- bis $C_8$-Cycloalkyl, Phenyl oder $C_7$- bis $C_{10}$-Phenalkyl bedeutet und die Cycloalkyl- und Phenylreste gegebenenfalls durch $C_1$- bis $C_4$-Alkyl substituiert sind,

$R^3$ und $R^4$ für Wasserstoff oder einer der Reste $R^3$ oder $R^4$ für $C_1$- bis $C_4$-Alkyl oder $C_7$- bis $C_9$-Phenalkyl und,

wenn n = 1 ist

X

a) für Phenyl, Naphthyl oder einen 5- oder 6-gliedrigen aromatischen gegebenenfalls mit einem Benzolring kondensierten Heterocyclus, wobei diese Reste gegebenenfalls durch $C_1$- bis $C_{12}$-Alkyl, $C_1$- bis $C_{12}$-Alkoxy, Methylendioxy, Ethylendioxy, Phenoxy, $C_7$- bis $C_9$-Phenylalkyloxy, $C_7$- bis $C_9$-Phenylalkyl, Trifluormethyl, Hydroxy, Halogen, $-S-R^7$, $-COR^7$, $-COOR^7$, -COOH, -CN, $-CONH-R^7$, $-NH-R^7$, $-N(R^7)_2$, $-O-COR^7$ oder $-NH-COR^7$ einfach oder zweifach substituiert sind, worin $R^7$ lineares oder verzweigtes $C_1$- bis $C_{12}$-Alkyl, $C_5$- bis $C_8$-Cycloalkyl, Phenyl oder $C_7$- bis $C_{10}$-Phenalkyl, worin die Cycloalkyl-und Phenylreste gegebenenfalls durch $C_1$- bis $C_4$-Alkyl substituiert sind, oder

wenn n = 2 ist

X

b) für Phenylen oder Naphthylen, wobei diese Reste gegebenenfalls durch $C_1$- bis $C_{12}$-Alkyl, $C_1$- bis $C_{12}$-Alkoxy oder Halogen substituiert sind, oder

c) für einen Rest der Formel

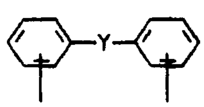

stehen, worin Y ein Rest der Formel $-(CH_2)_m$, -CO-, $-CO-(CH_2)_pCO-$, $-OCO-(CH_2)_pCOO-$, $-NH-CO-(CH_2)_p-CO-NH-$, -O-, $SO_2$-,

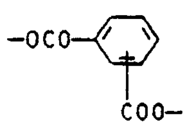 oder 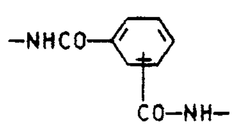 ,

m = 1, 2 oder 3 und p = 0 oder eine der Zahlen 1 bis 8 sind.

Die erfindungsgemäß gegen Lichteinwirkung stabilisierten Polymere zeigen im Vergleich zu den aus den US-PS 3 907 700 und 4 011 236 bekannten Verbindungen, die zu den erfindungsgemäß verwendeten als nächstliegender Stand anzusehen sind, überlegene Wirkung in der Langzeitstabilisierung eine geringe Flüchtigkeit, eine verringerte Migration und eine erhöhte Temperaturstabilität.

So erhält man z.B. mit der Verbindung des Beispiels 12 einen ausgezeichnet gegen den Abbau durch Licht stabilisierten Polyurethan-Integralschaum. Die hiermit erreichte Stabilisierung ist der durch Zusatz von Stabilisierungsmitteln des Standes der Technik wie 2-(2'-Hydroxy-5'-methylphenyl)-benztriazol, Sebacinsäure-di(1,2,2,6,6-Pentamethyl-4-piperidyl)ester und/oder Triethylenglykol-bis-3-(3-t-butyl-4-hydroxy-5-methylphenyl)-propionat erzielten deutlich überlegen.

Halogen bedeutet Fluor, vorzugsweise Chlor oder Brom.

Als Heteroarylreste kommen für $R^1$ und $R^2$ z.B. Pyridyl, Furyl, Chinolyl und Thiophenyl in Betracht.

Vorzugsweise stehen $R^1$ und $R^2$ für Phenyl, Pyridyl oder Furyl, wobei diese Reste gegebenenfalls durch $C_1$- bis $C_4$-Alkyl, $C_1$- bis $C_4$-Alkoxy, Chlor oder Brom substituiert sind.

Weiterhin sind Verbindungen (I) bevorzugt, in denen

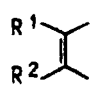

für einen ankondensierten Benzolring steht, wobei dieser Ring gegebenenfalls wie oben angegeben

substituiert ist.

Bevorzugte Substituenten am ankondensierten Benzolring sind Chlor, $C_1$ - bis $C_6$-Alkyl und $C_1$- bis $C_8$-Alkoxy wie Methyl, Ethyl, i-Propyl, 2-Butyl, tert.-Butyl, Amyl, Hexyl, Methoxy, Ethoxy, i-Propoxy, n-Butoxy, Pentoxy und Hexoxy.

$R^3$ und $R^4$ stehen für Wasserstoff oder einer der Reste $R^3$ oder $R^4$ für $C_1$- bis $C_4$-Alkyl oder $C_7$- bis $C_9$-Phenylalkyl wie Methyl, Ethyl, n-Propyl, n-Butyl, Benzyl, 2-Phenylethyl-1 oder 2-Phenylpropyl-2. Vorzugsweise sind $R^3$ und $R^4$ Wasserstoff.

Die Bedeutung von X ist davon abhängig, ob n = 1 oder 2 ist.

Ist n = 1, dann ist X Phenyl, Naphthyl oder ein 5- oder 6-gliedriger aromatischer - gegebenenfalls mit einem Benzolring kondensierter Heterocyclus, wobei die genannten Reste wie angegeben gegebenenfalls ein- oder zweifach substituiert sind. Von diesen Verbindungen (I) sind solche bevorzugt, bei denen X ein gegebenenfalls ein- oder zweifach substituiertes Phenyl ist.

Vorzugsweise steht X für durch lineares oder verzweigtes $C_1$- bis $C_{12}$-Alkyl, Trifluormethyl, $C_1$- bis $C_{12}$-Alkoxy, Methylendioxy, Ethylendioxy, $C_7$- bis $C_9$-Phenalkyl, Phenoxy, Phenyl, $C_1$- bis $C_{12}$-Alkoxycarbonyl, $C_1$- bis $C_{12}$-Alkylcarbonyl, Benzoyl, Cyano, N,N-Di-$C_1$- bis $C_6$-alkylamino, $C_1$- bis $C_{12}$-alkylcarbonylamino $C_1$- bis $C_{12}$-Alkanoyloxy, Benzoyloxy, $C_7$- bis $C_9$-Phenalkanoyloxy oder Chlor substituiertes Phenyl, wobei die Zahl der Substituenten 1 oder 2 ist.

Als Substituenten am Phenylrest X sind neben den bestimmt genannten im einzelnen z.B. zu nennen:

- α) Alkyl wie Methyl, Ethyl, n- und i-Propyl, n-Butyl, 2-Butyl, tert.-Butyl, n-Pentyl, tert.-Pentyl, n-Hexyl, n-Heptyl, n-Octyl, 2-Ethylhexyl, n-Nonyl, n-Decyl und n-Dodecyl;
- β) Alkoxy wie Methoxy, Ethoxy, i-Propoxy, n-Butoxy, n-Pentoxy, i-Pentoxy, n-Hexoxy, n-Heptoxy, n-Octoxy, 2-Ethylhexoxy, n-Nonoxy, n-Decoxy und n-Dodecoxy;
- γ) Phenoxy, Benzyloxy, 2-Phenylethoxy, 3-Phenylpropoxy.

Besonders bevorzugt sind Verbindungen (I) mit n = 1, bei denen X für 4-$C_1$- bis $C_{12}$-Alkylphenyl, 4-$C_1$- bis $C_{12}$-Alkoxyphenyl, 4-Phenoxyphenyl, 4-$C_1$- bis $C_{12}$-Alkylcarbonylaminophenyl oder 4-$C_1$- bis $C_{12}$-Alkanoyloxyphenyl steht.

Besonders bevorzugt sind somit Verbindungen der allgemeinen Formel (II)

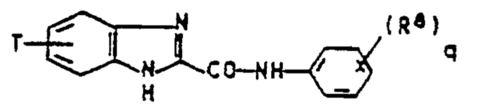 (II),

in der

- T für Wasserstoff, $C_1$- bis $C_4$-Alkyl, $C_1$- bis $C_4$-Alkoxy, Trifluormethyl, Phenyl, $C_7$- bis $C_9$-Phenylalkyl oder Chlor,
- $R^8$ für $C_1$- bis $C_{12}$-Alkyl, $C_1$- bis $C_{12}$-Alkoxy, Phenoxy, $C_7$- bis $C_9$-Phenalkoxy, $C_7$- bis $C_9$-Phenalkyl, Trifluormethyl, N,N-Di-$C_1$- bis $C_6$-alkylamino, $C_2$- bis $C_{12}$-Alkanoylamino, $C_2$- bis $C_{12}$-Alkanoyloxy, Benzoyloxy, $C_7$- bis $C_9$-Phenalkanoyloxy, $C_1$- bis $C_{12}$-Alkylcarbonyl, $C_1$- bis $C_{12}$-Alkoxycarbonyl, Benzoyl, Cyano, Methylendioxy oder Ethylendioxy und
- q für 1 oder 2 stehen.

Von den Verbindungen der allgemeinen Formel (II) sind wieder von besonderem Interesse solche, bei denen

- T Wasserstoff, Methyl oder Chlor,
- $R^8$ $C_1$- bis $C_{12}$-Alkyl, $C_1$- bis $C_{12}$-Alkoxy, Methylendioxy, Ethylendioxy, Trifluormethyl oder $C_2$- bis $C_{12}$-Alkanoylamino und
- q 1 oder 2 bedeuten.

Ist n = 2, dann ist X entweder (b) ein gegebenenfalls durch Alkyl oder Alkoxy substituiertes Phenylen oder Naphthylen oder (c) ein Rest der Formel

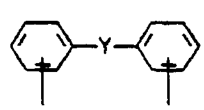

worin Y die oben angegebene Bedeutung hat. Vorzugsweise ist Y $-CH_2-$, -O-oder $-SO_2-$.

Bei n = 2 sind Verbindungen (1) bevorzugt, bei denen X gegebenenfalls durch $C_1$- bis $C_{12}$-Alkyl oder $C_1$- bis $C_{12}$-Alkoxy substituiertes Phenylen oder ein Rest der Formel II ist, wobei die Phenylenreste in (II) gegebenenfalls durch $C_1$- bis $C_4$-Alkyl oder $C_1$- bis $C_4$-Alkoxy, vorzugsweise jedoch unsubstituiert sind und Y $-CH_2-$ oder -O- bedeutet.

Die Erfindung betrifft außerdem neue Imidazol-2-carbonsäureanilide der allgemeinen Formel (Ia)

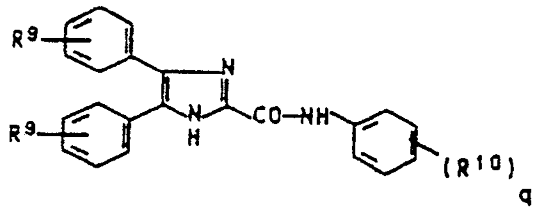

(Ia)

in der

| | |
|---|---|
| $R^9$ | Methyl, Methoxy oder Wasserstoff, |
| $R^{10}$ | $C_1$- bis $C_{12}$-Alkyl, $C_1$- bis $C_{12}$-Alkoxy, Chlor und |
| q | 1 oder 2 bedeuten. |

Weiterhin betrifft die Erfindung neue Imidazol-2-carbonsäureanilide der allgemeinen Formel (Ib)

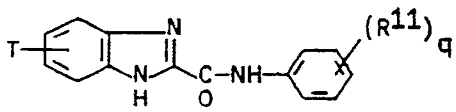

(Ib)

in der

T für Wasserstoff, q für 1 und $R^{11}$ für 4'-$CH_3$, 4' -$C_2H_5$, 2'-$C_2H_5$, 4'-i-$C_3H_7$, 4'-t-$C_4H_9$, 2'-$OC_2H_5$, 4'-$OC_2H_5$, 3'-$OC_2H_5$, 4'-$OC_6H_{13}$-n, 4' -$OC_8H_{17}$-n, 4'-$OC_{12}H_{25}$-n, 4'-$C_{10}H_{21}$-n, 4'-$C_{12}H_{25}$-n, 4'-$N(CH_3)_2$, 3'-$CF_3$, 2'-$OC_4H_9$-n, 2'-$OC_6H_{13}$-n, 2'-$OC_8H_{17}$-n, 2'-$OC_{12}H_{25}$-n, 4'-$OC_6H_5$,4'-$COC_6H_5$, 2'-$COC_6H_5$, 4'-CN, 4'-$C_4H_9$-n, 4'-NH-CO-$CH_2CH_3$, 4'-NH-CO-t-$C_4H_9$, 4'-NH-CO-$CH(C_2H_5)C_4H_9$-n, 4'-$OC_5H_{11}$-n, 4'-$OC_5H_{11}$-i, 4'-$OC_7H_{15}$-n, 4'-$OCH_2$-$CH(C_2H_5)C_4H_9$-n, 4'-$OC_9H_{19}$-n, 4'-$OC_{10}H_{21}$-n, 4'-$OCH_2$-$C_6H_5$, 4'-$OCH_2$-$CH_2$-$C_6H_5$, 4'-O-CO-$C(CH_3)_3$, 4'-O-CO-$C_2H_5$, 4'-O-CO-$CH(C_2H_5)C_4H_9$-n oder 4'-O-CO-$C_9H_{19}$ oder

T für 5- oder 6-$CH_3$, q für 1 und $R^{11}$ für 4'-t-$C_4H_9$, 4'-i-$C_3H_7$ oder4'-$CO_2C_2H_5$ oder

T für 5- oder 6-$CH_3$, q für 2 und $R^{11}$ für 2',5'-$(OC_2H_5)_2$ oder

T für 4-, 5-, 6- oder 7-$OC_2H_5$ oder für 4-, 5-, 6-, 7-$C(CH_3)_2$-$C_6H_5$, q für 1 und $R^{11}$ für 4'-$OC_2H_5$ oder

T für Wasserstoff, q für 2 und $R^{11}$ für 2',5'-$(OC_2H_5)_2$ 3'4'-O-$CH_2$-O-, 3',4'-O-$(CH_2)_2$-O-, 3',4'-$(OCH_3)_2$, 2',6'-$(CH_3)_2$ oder 2',6'-$[CH(CH_3)_2]_2$ steht.

Von den Imidazol-2-carbonsäureaniliden der allgemeinen Formel (Ib) werden solche bevorzugt, bei denen T für Wasserstoff, q für 1 und $R^{11}$ für 4'-$C_2H_5$, 2'-$C_2H_5$, 4'-i-$C_3H_7$, 4'-$OC_2H_5$, 3'-$OC_2H_5$, 4'-$OC_6H_{13}$-n, 4'-$OC_8H_{17}$-n, 4'-$C_{12}H_{25}$-n oder 4'-$C_4H_9$-n steht.

Die neuen Verbindungen der Formeln (Ia) und (Ib), in denen $R^{10}$ oder $R^{11}$ lineares $C_8$- bis $C_{12}$-Alkoxy bedeuten, sind hervorragend zur Stabilisierung von Überzügen, wie Lackierungen, und Zweischicht-Metallic-Lackierungen geeignet. Durch den Zusatz dieser Stabilisatoren wird die Gebrauchsechtheit, insbesondere wird die Licht- und Wetterechtheit von pigmentierten Industrielackierungen deutlich verbessert.

Die Verbindungen der Formeln (I), (II), (Ia) und (Ib) Können nach bekannten bzw. an sich bekannten Verfahren hergestellt werden.

Wenn in (I) $R^1$ und $R^2$ einen ankondensierten Benzolring bedeuten, können diese Verbindungen durch Kondensation eines o-Phenylendiaminderivates der Formel (III)

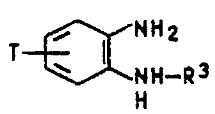

(III)

mit einem Oxalsäureethylester-mono-amid der Formel (IV)

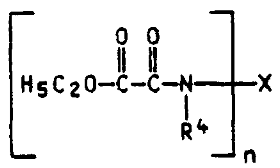

(IV)

nach dem in der FR-PS 1 517 719 bzw. in Bull. Soc. Chim., 10, 3368 (1966) angegebenen Verfahren synthetisiert werden.

Eine weitere Synthesemöglichkeit besteht in der Umsetzung von Benzimidazol-2-carbonsäurederivaten mit aromatischen Aminen:

i) wenn $R^8$ für einen elektronenziehenden Substituenten steht, durch Umsetzen des 2-Trichlorbenzimidazolderivates (V)

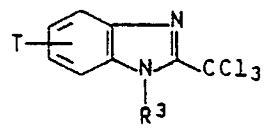

(V)

mit einem aromatischen Amin der Formel (VI)

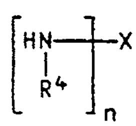

(VI)

nach J. Chem. Soc. Sect. C., 1967, 20, und

ii) wenn $R^8$ einen Elektronendonorsubstituenten bedeutet, durch Reaktion eines Dibenzoimidazo-(1,2-a,1',2'-d)-tetrahydropyrazin-6,13-dionderivatesder Formel (VII)

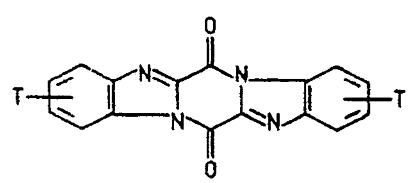

(VII)

mit einem aromatischen Amin der Formel VIII, analog der in Indian J. Chem., 188, 464 (1979) beschriebenen Umsetzung.

Weiterhin sind die Verbindungen der Formel (I) durch Umsetzen eines Imidazolderivates der Formel (VIII)

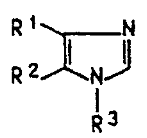

(VIII)

mit einem aromatischen Isocyanat der Formel (IX)

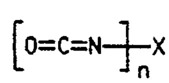

(IX)

gut zugänglich. Die Reaktion erfolgt analog Chem. Ber. 92, 550 (1959).

Die Verbindungen (I), (II), (Ia) und (Ib) werden nach üblichen Verfahren in die Polymeren eingearbeitet.

Die Einarbeitung kann beispielsweise durch Einmischen der Verbindungen und gegebenenfalls weiterer Additive in die Schmelze nach der in der Technik üblichen Methode vor oder während der Formgebung, oder auch durch Aufbringen der gelösten oder dispergierten Verbindungen auf das Polymere direkt oder durch Einmischen in eine Lösung, Suspension oder Emulsion des Polymeren, gegebenenfalls unter nachträglichem Verdunstenlassen des Lösungsmittels, erfolgen.

Die erfindungsgemäßen Stabilisatoren werden einzeln oder als Gemisch den Polymeren in Mengen von 0,01 bis 5, vorzugsweise von 0,05 bis 2,5 und insbesondere von 0,1 bis 2 Gewichtsprozent, bezogen auf das zu stabilisierende Material, zugemischt.

Die so stabilisierten Materialien können in die für die Anwendung üblichen Formen überführt werden, z.B. Folien, Fasern, Bändchen, Profile oder als Bindemittel für Lacke, Klebstoffe, Kitte oder Formmassen.

In der Praxis können die Verbindungen der Formel I zusammen mit 0,1 bis 5, vorzugsweise 0,5 bis 3 Gew.% weiteren üblichen Zusatzstoffen, wie Antioxidantien, weitere Lichtstabilisatoren, oder Gemische davon angewendet werden.

Solche üblichen Zusatzstoffe sind z.B.: Antioxidantien, UV-Absorber und Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, außerdem Nickelverbindungen, sterisch gehinderte Amine, Metalldesaktivatoren, Phosphite, peroxidzerstörende Verbindungen, Polyamidstabilisatoren, basische Co-Stabilisatoren, Nukleierungsmittel und sonstige Zusätze wie Weichmacher, Gleitmittel, Emulgatoren, Füllstoffe, Ruß, Kaolin, Talk, Glasfasern, Pigmente, optische Aufheller, Flammschutzmittel und Antistatica.

Die Verbindungen der Formel (I) eignen sich insbesondere zum Schutz von Polyurethanen, die sich von Polyethern, Polyestern und Polybutadienen mit endständigen Hydroxylgruppen und aliphatischen oder aromatischen Polyisocyanaten ableiten, sowie deren Vorprodukte gegen Abbau durch Wärme-und insbesondere durch Lichteinwirkung.

Eine verbesserte stabilisierende Wirkung erzielt man, wenn man zusätzlich ein bekanntes Antioxidanz, z.B. eine Verbindung auf der Basis sterisch gehinderter Phenole oder einen Schwefel oder Phosphor enthaltenden Costabilisator verwendet.

Als derartige phenolische Antioxidationsmittel kommen z.B. 2,6-Di-tert.-butyl-4-methylphenol, n-Octadecyl-β-(3,5-di-tert.-butyl-4-hydroxyphenyl)-propionat, 1,1,3-Tris-(2'-methyl-4'-hydroxy-5'-tert.-butyl-phenyl)-butan, 1,3,5-Trimethyl-2,4,6-tris-(3',5'-di-tert.-butyl-4'-hydroxy-benzyl)-benzol, 1,3,5-Tris-(3',5'-di-tert.-butyl-4'-hydroxybenzyl)-isocyanurat, 1,3,5-Tris-(3',5'-di-tert.-butyl-4'-hydroxyphenyl)-propionyloxy-ethyl]-isocyanurat, 1,3,5-Tris-(2',6'-di-methyl-3'-hydroxy-4'-tert.-butylbenzyl)-isocyanurat, Pentaerythrit-tetrakis-[β-(3,5-di-tert.-butyl-4-hydroxyphenyl)-propionat], Triethylenglykol-bis-3-(3'-t-butyl-4'-hydroxy-5'-methylphenyl)-propionat, Derivate des 6-Hydroxy-2,5,7,8-tetramethylchromans wie α-Tocopherol und Stearinsäure-(6-hydroxy-2,5,7,8-tetramethylchroman-2-yl)-ethylester in Betracht.

Als phosphorhaltige Antioxidantien sind z.B. zu nennen: Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit.

Als Schwefel enthaltende Antioxidationsmittel sind z.B. Dilaurylthiodipropionat, Dimyristylthiodipropionat, Distearylthiodipropionat, Pentaerythrittetrakis-(β-laurylthiopropionat), Pentaaerythrittetrakis-(β-hexylthiopropionat) zu nennen.

Eine besonders gute Stabilisierung erhält man, wenn zu den Verbindungen der Formeln (I), (II), (Ia) und (Ib) noch mindestens einen Lichtstabilisator aus der Verbindungsklasse der sterisch gehinderten Amine in üblicher Konzentration zusetzt.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, Bis-(1,2,2,6,6-pentamethylpiperidyl)-ester, das Kondensationsprodukt von 1-Hydroxyethyl-2,2,6,6-tetramethyl-4-hydroxypiperidin und Bernsteinsäure, das Kondensationsprodukt von N,N'-(2,2,6,6-Tetramethylpiperidyl)-hexamethylendiamin und 4-tert.-Octylamino-2,6-dichlor-1,3,5-s-triazin, Tris-(2,2,6,6-Tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Eine ganz besonders gute Stabilisierung von Polyurethanen erhält man, wenn das Polyurethan ein Gemisch aus mindestens einer Verbindung der Formel (I), mindestens einem der oben genannten Antioxidantien und mindestens einer Verbindung eines sterisch gehinderten Amins enthält. Die folgenden Beispiele sollen die Erfindung zusätzlich erläutern.

A) Herstellungsbeispiele

Beispiel 1

20,7 g (0,1 mol) Oxalsäureethylester-(4-methyl-phenyl)-amid und 10,8 g (0,1 mol) o-Phenylendiamin werden in 50 ml trockenem Dimethylformamid unter Stickstoff 8 h auf Rückflußtemperatur erwärmt. Nach dem Abkühlen versetzt man das Reaktionsgemisch mit 200 ml Methanol und 140 g Eis. Der ausgefallene Feststoff wird abgesaugt und mit einem Methanol-Wasser-Gemisch (1:1) gewaschen. Umkristallisation aus Ethanol unter Zusatz von Aktivkohle liefert 9,5 g farbloses Produkt der Formel

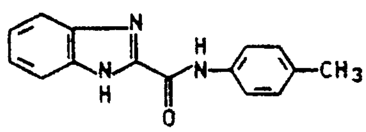

mit Schmp. 226-227° C.
Analyse: $C_{15}H_{13}N_3O$ (251,3)
ber.: C 71,69 H 5,21 N 16,72 O 6,37
gef.: C 71,6 H 5,5 N 16,2 O 7,0

Beispiele 2 bis 11

Analog Beispiel 1 erhält man mit den entsprechenden Ausgangsprodukten die in der Tabelle 1 aufgeführten Verbindungen.

Tabelle 1

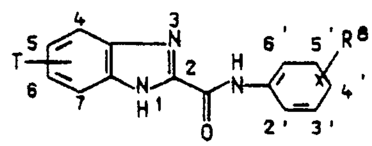

| Beispiel | T | $R^8$ | Schmp. [°C] |
|---|---|---|---|
| 2 | H | $4'-C_2H_5$ | 219 |
| 3 | H | $2'-C_2H_5$ | 200 |
| 4 | H | $4'-i-C_3H_7$ | 222 |
| 5 | H | $4'-t-C_4H_9$ | 235 |
| 6 | H | $2',5'-(t-C_4H_9)_2$ | 224-226 |
| 7 | H | $2'-OC_2H_5$ | 219-220 |
| 8 | $5/6-CH_3$ *) | $4'-t-C_4H_9$ | 211-213 |
| 9 | $5/6-CH_3$ *) | $4'-i-C_3H_7$ | 247-249 |
| 10 | $5/6-CH_3$ *) | $2',5'-(OC_2H_5)_2$ | 212-215 |
| 11 | $5/6-CH_3$ *) | $4'-CO_2C_2H_5$ | 207-208 |

*) Gemisch aus 5- und 6-Methylverbindung

Beispiel 12

14,4 g (0,05 mol) Dibenzimidazo-(1,2a,1',2'-d)-tetrahydropyrazin-6,13-dion und 14,8 g (0,11 mol) p-Phenetidin werden in 80 ml Dimethylformamid 3,5 h unter Rückfluß erhitzt. Nach Verdünnen mit 300 ml Methanol wird das ausgefallene Produkt abgesaugt und mit Methanol gewaschen. Man erhält 18,0 g des Benzimidazol-2-carboxanilides der Formel

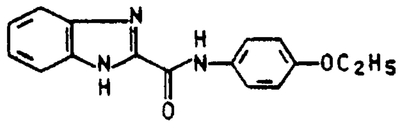

Schmp. 201-202 °C.
Analyse: $C_{16}H_{15}N_3O_2$ (281,21)
ber.: C 68,31 H 5,37 N 14,94 O 11,37
gef.: C 68,2 H 5,5 N 14,9 O 11,3

Beispiele 13 bis 31

Analog Beispiel 12 erhält man mit den entsprechenden Ausgangsprodukten die in der Tabelle 2 genannten Verbindungen.

Tabelle 2

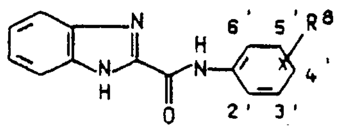

| Beispiel | $R^8$ | Schmp. [°C] |
|---|---|---|
| 13 | 4'-$OCH_3$ | 237-239 |
| 14 | 3'-$OC_2H_5$ | 211-213 |
| 15 | 4'-$OC_4H_9$-n | 163-165 |
| 16 | 4'-$OC_6H_{13}$-n | 151-153 |
| 17 | 4'-$OC_8H_{17}$-n | 143-145 |
| 18 | 4'-$OC_{12}H_{25}$-n | 141-142 |
| 19 | 2',5'-$(OC_2H_5)_2$ | 243-244 |
| 20 | 3',4'-O-$CH_2$-O | 262-264 |
| 21 | 3',4'-O-$(CH_2)_2$-O- | 268-270 |
| 22 | 4'-$C_{10}H_{21}$-n | 140-141 |
| 23 | 4'-$C_{12}H_{25}$-n | 130-131 |
| 24 | 4'-$N(CH_3)_2$ | 253-254 |
| 25 | 3'-$CF_3$ | 203 |
| 26 | 4'-$NHCOCH_3$ | 308 |
| 27 | 2'-$OC_4H_9$-n | 183-184 |
| 28 | 2'-$OC_6H_{13}$-n | 175-176 |
| 29 | 2'-$OC_8H_{17}$-n | 160-163 |
| 30 | 2'-$OC_{12}H_{25}$-n | 145-146 |
| 31 | 4'-$OC_6H_5$ | 237-239 |
| 32 | 3',4'-$(OCH_3)_2$ | 209 |

Beispiel 33

23,6 g (0,1 mol) 2-Trichlormethylbenzimidazol und 19,7 g (0,1 mol) 4-Aminobenzophenon werden in 220 g 14 %iger Salzsäure 5 h zum Rückfluß erhitzt. Nach dem Abkühlen wird das ausgefallene Produkt abgesaugt und mit Aceton gewaschen. Der Rückstand wird in 800 ml Ethanol und 50 ml 20 %iger Natronlauge gelöst und anschließend die Lösung auf 2 l Eiswasser gegossen. Der ausgefallene Feststoff wird abgesaugt und mit Wasser neutral gewaschen. Nach dem Trocknen erhält man 17,6 g farbloses Produkt der Formel

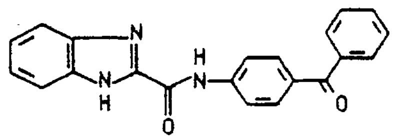

Schmp. 230-231 ° C.
Analyse:
$C_{21}H_{15}N_3O_2$ (341,37)
ber.: C 73,89 H 4,42 N 12,31 O 9,37
gef.: C 73,9 H 4,3 N 12,1 O 9,4

Beispiele 34 bis 37

Die in Tabelle 3 angegebenen Verbindungen wurden analog Beispiel 33 hergestellt.

Tabelle 3

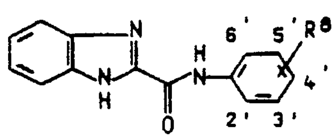

| Beispiel | $R^8$ | Schmp. [°C] |
|---|---|---|
| 34 | 2'-$COC_6H_5$ | 250-252 |
| 35 | 4'-$CO_2C_2H_5$ | 214-215 |
| 36 | 4'-CN | 302-303 |
| 37 | 2',4'-$(Cl)_2$ | 304 |

Beispiel 38

11,8 g (0,1 mol) Benzimidazol und 21 g (0,12 mol) 4-n-Butylphenylisocyanat werden in 180 ml trockenem Nitrobenzol 6 h auf Rückflußtemperatur erwärmt. Der nach dem Abkühlen ausgefallene Niederschlag wird abgesaugt und mit Nitrobenzol und Methanol gewaschen. Man erhält 20,5 g eines farblosen Feststoffes der Formel

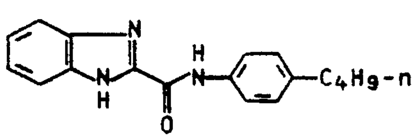

Schmp. 205-206 ° C.
Analyse: $C_{18}H_{19}N_3O$ (293,4)

ber.: C 73,69 H 6,53 N 14,32 O 5,45
gef.: C 73,9 H 6,8 N 14,1 O 5,5

Beispiele 39 bis 71

Analog Beispiel 38 wurden aus den in Tabelle 4 angegebenen Imidazolen und Isocyanaten die entsprechenden Imidazol-2-carbonsäureanilide hergestellt.

Tabelle 4

| Beispiel | Imidazolderivat | Isocyanat | Schmp. [°C] |
|---|---|---|---|
| 39 | 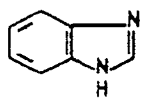 | 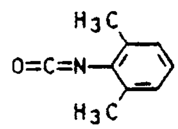 | 230-231 |
| 40 | 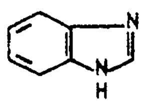 | 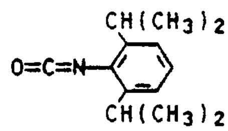 | 264-265 |
| 41 | 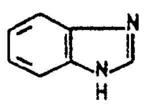 | 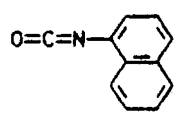 | 282-285 |
| 42 | 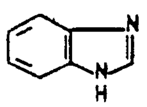 | 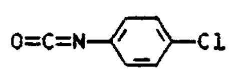 | 248-250 |
| 43 | 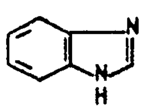 | 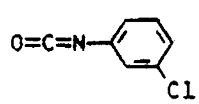 | 244 |
| 44 | 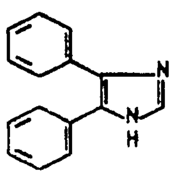 | 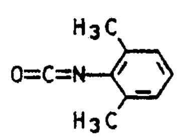 | 264 |
| 45 | 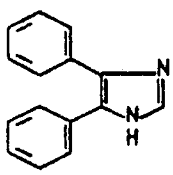 | 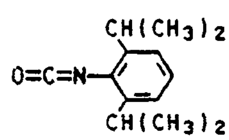 | 290-292 |

| Beispiel | Imidazolderivat | Isocyanat | Schmp. [°C] |
|---|---|---|---|
| 46 | 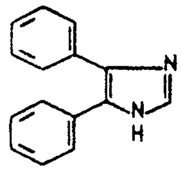 | 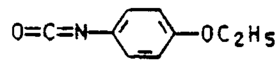 | 223 |
| 47 | 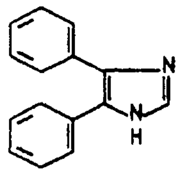 | 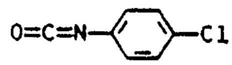 | 237-239 |
| 48 | 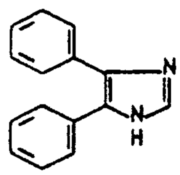 | 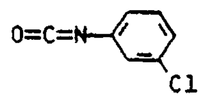 | 186-189 |
| 49 | 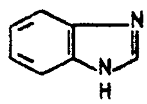 | 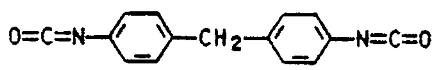 | 327 |
| 50 | 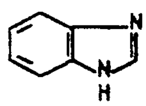 | 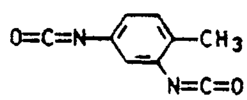 | 350 (Zers.) |
| 51 | 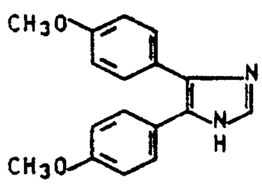 | 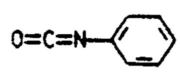 | 236-238 |
| 52 | 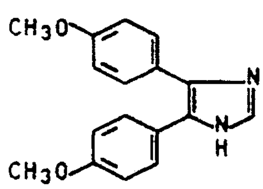 | 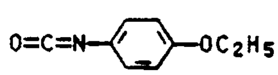 | 197-198 |

| Beispiel | Imidazolderivat | Isocyanat | Schmp. [°C] |
|---|---|---|---|
| 53 | 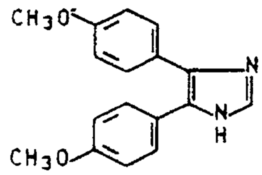 | 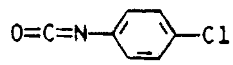 | 233-235 |
| 54 | 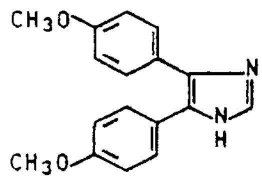 | 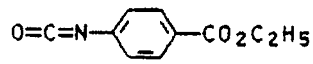 | 237-240 |
| 55 | 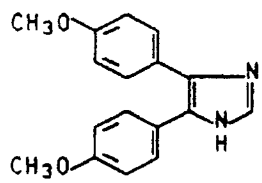 | 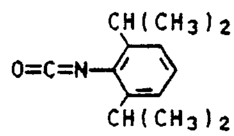 | 244-246 |
| 56 | 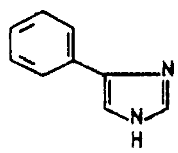 | 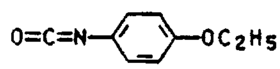 | 158-160 |
| 57 | 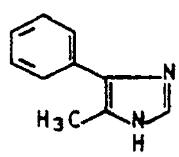 | 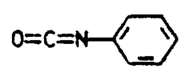 | 217-218 |
| 58 | 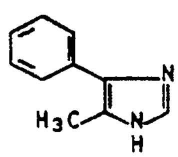 | 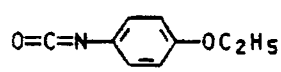 | 192 |
| 59 | 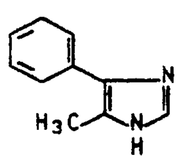 | 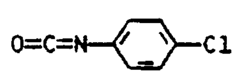 | 238-240 |

| Beispiel | Imidazolderivat | Isocyanat | Schmp. [°C] |
|---|---|---|---|
| 60 | 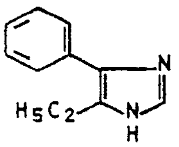 | 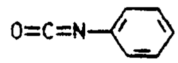 | 157-158 |
| 61 | 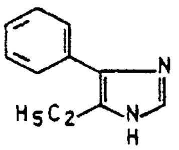 | 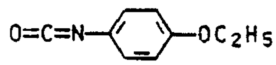 | 170-172 |
| 62 | 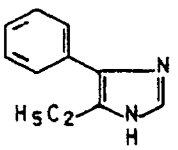 | 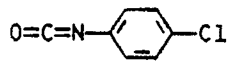 | 180-181 |
| 63 | 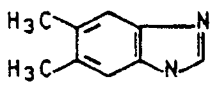 | 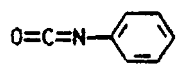 | 299 |
| 64 | 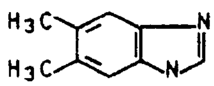 | 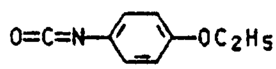 | 272 |
| 65 | 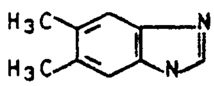 | 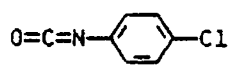 | 286 |
| 66 | 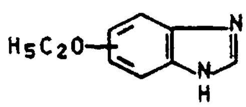 | 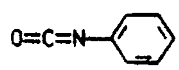 | 210-212 |
| 67 | 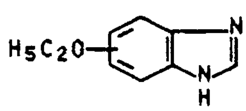 | 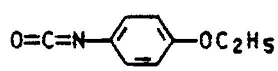 | 212 |
| 68 | 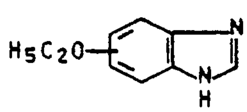 | 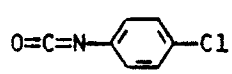 | 228-229 |

| Beispiel | Imidazolderivat | Isocyanat | Schmp. [°C] |
|---|---|---|---|
| 69 | 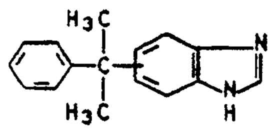 | 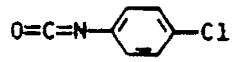 | 103 |
| 70 | 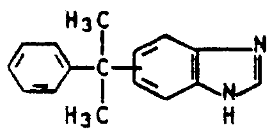 | 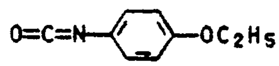 | 207-209 |
| 71 | 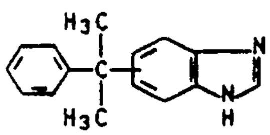 | 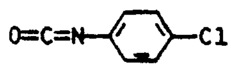 | 205-206 |

Beispiel 72

14,k g (0,05 mol) Dibenzimidazo-(1,2a,1',2'-d)-tetrahydropyrazin-6,13-dion und 30 g (0,18 mol) 4-Propionylaminoanilin werden in 70 ml trockenem Dimethylformamid 3 h unter Rückfluß erhitzt. Das Reaktionsgemisch wird nach dem Abkühlen mit etwa 700 ml Methanol versetzt. Der ausgefallene Feststoff wird abgesaugt, mit Methanol gewaschen und getrocknet. Man erhält 20,7 g des Benzimidazol-2-carboxanilids der Formel

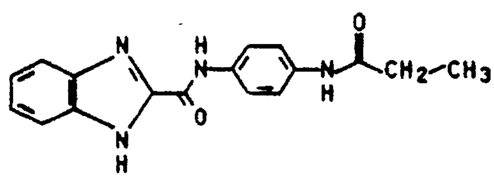

Schmp. 289-291 °C
Analyse: $C_{17}H_{18}N_4O_2$ (308,34)
ber.: C 66,22 H 5,23 N 18,17 O 10,38
gef.: C 66,4 H 5,3 N 17,8 O 10,5

Beispiele 73 bis 84

Analog Beispiel 72 erhält man aus den entsprechenden Ausgangsprodukten die in Tabelle 5 angegebenen Verbindungen.

Tabelle 5

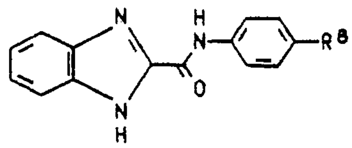

| Beispiel | $R^8$ | Schmp. [°C] |
|---|---|---|
| 73 | $NH-CO-t-C_4H_9$ | 258-260 |
| 74 | $NH-CO-CH(C_2H_5)C_4H_9-n$ | 273-274 |
| 75 | NH-CO-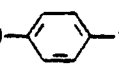-t-$C_4H_9$ | 311-313 |
| 76 | $OC_5H_{11}-n$ | 200 |
| 77 | $OC_5H_{11}-i$ | 220 |
| 78 | $OC_7H_{15}-n$ | 160 |
| 79 | $OCH_2-CH(C_2H_5)C_4H_9-n$ | 140 |
| 80 | $OC_9H_{19}-n$ | 150 |
| 81 | $OC_{10}H_{21}-n$ | 145 |
| 82 | $OCH_2-C_6H_5$ | 217 |
| 83 | $OCH_2-CH_2-C_6H_5$ | 194 |
| 84 | OH | 328-330 |

Beispiel 85

Zu einer Lösung von 12,7 g (0,05 mol) 4'-Hydroxy-benzimidazol-2-carboxanilid(aus Beispiel 78) in 50 ml trockenem Pyridin werden bei 25 bis 50 °C 7,8 g (0,065 mol) Pivalinsäurechlorid zugetropft und 3 h bei 50 °C gerührt. Die Reaktionslösung wird auf 300 ml Eiswasser und 100 ml konz. Salzsäure gegossen und 20 min gerührt. Der ausgefallene Feststoff wird abgesaugt und mit Wasser gewaschen. Umkristallisation aus Toluol liefert 9,5 g einer farblosen Produktes des Formel

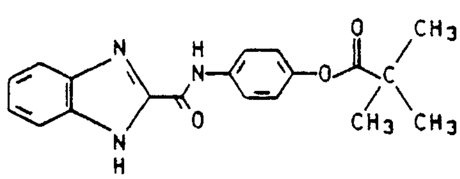

Schmp. 266-267 °C

Analyse: $C_{19}H_{19}N_3O_3$ (337,38)

ber.: C 67,64 H 5,68 N 12,45 O 14,22

gef.: C 67,8 H 5,8 N 12,2 O 14,1

Beispiele 86 bis 89

Analog Beispiel 85 werden folgende, in Tabelle 6 aufgeführte Verbindungen hergestellt.

Tabelle 6

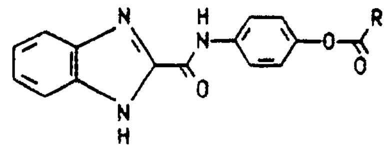

| Beispiel | R | Schmp. [°C] |
|---|---|---|
| 86 | $C_2H_5$ | 227-229 |
| 87 | $CH(C_2H_5)C_4H_9$-n | 154 |
| 88 | $C_9H_{19}$ (Isomerengem.) | 135-137 |
| 89 | 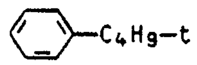 | 281-283 |

Beispiel 90

Zu einer Suspension von 8,45 g (0,03 mol) 4'-Ethoxy-benzimidazol-2-carboxanilid (aus Beispiel 12) und 2,3 g Kaliumhydroxid in 90 ml wasserfreiem Ethanol werden innerhalb 30 Min. bei Raumtemperatur 9,0 g Methyljodid getropft. Oie Reaktionsmischung wird 24 h bei Raumtemperatur gerührt, auf 300 ml Wasser gegeben, abgesaugt und mit Wasser gut gewaschen. Es werden 8.2 g eines farblosen Produktes der Formel

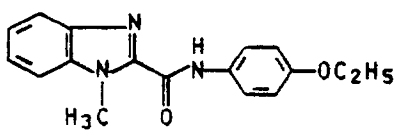

mit dem Schmp. 151 °C isoliert.
Analyse: $C_{17}H_{17}N_3O_2$ (295,34)
ber.: C 69.15 H 5.77 N 14.24 O 10.84
gef. : C 69.3 H 5.8 N 14.3 O 10.8

Beispiel 91

Zu einer Lösung von 14.1 g (0.05 mol) 4'-Ethoxy-benzimidazol-2-carboxanilid (aus Beispiel 12) in 250 ml trockenem Dimethylformamid werden 5,5 g Kaliumhydrogencarbonat und 7,1 g 1-Brompropan zugegeben und 16 h bei 80 bis 85 °C gerührt. Die Reaktionsmischung wird heiß abfiltriert mit 20 bis 30 ml Dimethylformamid gewaschen und das Lösungsmittel am Rotationsverdampfer abgezogen. Der Rückstand wird in 100 ml Methanol in der Hitze gelöst, mit Aktivkohle versetzt, abfiltriert. Beim Abkühlen kristallisieren 12,7 g eines farblosen Feststoffes der Formel

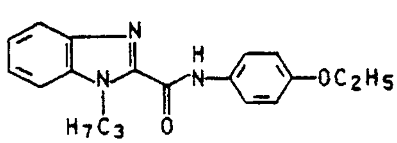

Schmp. 96 - 97 °C
Analyse: $C_{19}H_{21}H_3O_2$ (323,40)

ber.: C 70.59 H 6.50 N 13.0 O 9.91
gef.: C 70.1 H 6.7 N 12.9 O 10.0

Beispiel 92

Analog zu Beispiel 91 erhält man bei Verwendung von Benzylbromid an Stelle von 1-Brompropan das folgende Produkt mit der Formel

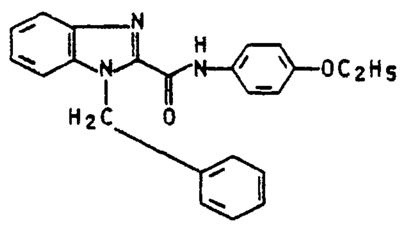

Schmp. 157 - 159 °C
Analyse: $C_{23}H_{21}N_3O_2$ (371.44)
ber. C 74.39 H 5.67 N 11.32 O 8,62
gef.: C 74.1 H 5.7 N 11.5 O 9.0

B) Anwendungsbeispiele:

Beispiel 93 (Lichtstabilisierende Wirkung in Polyurethan)

Die in Tabelle 7 aufgeführten Stabilisatoren werden in 150 g Polyetherol bei 40 - 50 °C unter Rühren gelöst. Die Lösung wird mit 76,5 g einer Diphenylmethandiisocyanatrezeptur 10 bis 15 Sekunden mit einem Rührwerk (1000 U/min innig vermischt. Das Gemisch wird sofort in eine auf 50°C vorgeheizte verschließbare Form gegossen und in verschlossenem Zustand 3 bis 4 Minuten aushärten lassen.

Für die Prüfung werden Schaumabschnitte mit geschlossener Oberfläche verwendet. Die Proben werden im Xenotest® 1200 belichtet und an den Proben der Yellowness Index (YI) gemäß ASTM D 1925 bestimmt. Die Ergebnisse sind in Tabelle 7, Spalten 3 bis 6, angegeben.

Tabelle 7

Cyclus im Xenotest 1200: 17 min Belichtung
3 min Beregnung
Temp. 45°C

| Stabilisator | Konzentration [%] | Y I nach A S T M D 1925 0 h | 24 h | 48 h | 72 h |
|---|---|---|---|---|---|
| 7.1 Kontrolle | - | 10,0 | 55,0 | 66,0 | 73,0 |
| 7.2 Verbindung aus Bsp. 3 | 1,0 | 15,3 | 40,4 | 52,6 | 65,0 |
| 7.3 Verbindung aus Bsp. 2 | 1,0 | 13,9 | 37,2 | 46,8 | 56,4 |
| 7.4 Verbindung aus Bsp. 4 | 1,0 | 14,4 | 34,2 | 43,5 | 54,8 |
| 7.5 Verbindung aus Bsp. 12 | 1,0 | 11,5 | 27,5 | 38,2 | 50,7 |
| 7.6 Verbindung aus Bsp. 12<br>+ A*)<br>+ B**) | 0,5<br>0,5<br>0,5 | 10,0 | 30,1 | 37,2 | 46,8 |
| 7.7 Verbindung aus Bsp. 12<br>+ A*)<br>Triethylenglykol-bis-3--(3-t-butyl-4-hydroxy-5--methylphenyl)-propionat | 0,5<br>0,5<br>0,5 | 11,0 | 26,5 | 29,5 | 41,5 |
| 7.8 2-(2'-Hydroxy-5'-methyl-phenyl)-benztriazol<br>+C***<br>+Triethylenglykol-bis-3--(3-t-butyl-4-hydroxy-5--methylphenyl)-propionat | 0,5<br>0,5<br>0,5 | 12,2 | 32,2 | 42,0 | 48,5 |

A*) = 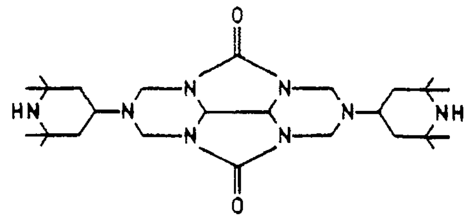

B**) = Gemisch aus 1 Gew.-Teil α-Tocopherol + 10 Gew.-Teile Tris(nonylphenyl)phosphit

C*** = 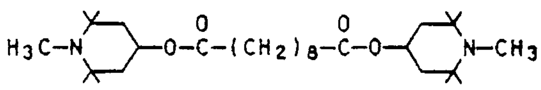

Beispiel 94

Die folgenden in Tabelle 8 aufgeführten Proben, die analog zu der in Beispiel 93 beschriebenen Methode präparariert wurden, wurden 48 h im Xenotest ®450 belichtet und die Yellowness-Index-Werte nach ASTM D 1925 bestimmt.

Tabelle 8

| | Stabilisator | Konz. (Gew.%) | Yellowness-Index nach ASTM D 1925 im Xenotest 450 nach 0 h | 48 h |
|---|---|---|---|---|
| 8.1 | Kontrolle | - | 1,72 | 47,91 |
| 8.2 | Verbindung aus Bsp. 13 | 1 | 5,79 | 20,81 |
| 8.3 | Verbindung aus Bsp. 12 | 1 | 1,91 | 18,23 |
| 8.4 | Verbindung aus Bsp. 15 | 1 | 3,59 | 19,15 |
| 8.5 | Verbindung aus Bsp. 16 | 1 | 2,89 | 12,88 |
| 8.6 | Verbindung aus Bsp. 17 | 1 | 5,11 | 21,11 |
| 8.7 | Verbindung aus Bsp. 14 | 1 | 10,04 | 26,76 |
| 8.8 | Verbindung aus Bsp. 23 | 1 | 6,58 | 21,68 |

Beispiel 95

Analog zu Beispiel 94 wurden folgende in Tabelle 9 aufgelisteten Stabilisatorkombinationen in PUR-Schaumsystemen geprüft.

Tabelle 9: Xenotest ®450

| | Stabilisator-Mischung | Konz. (Gew.%) | Yellowness-Index nach ASTM D 1925 im Xenotest 450 nach 0 h | 48 h |
|---|---|---|---|---|
| 9.1 | Kontrolle | - | 3,84 | 24,77 |
| 9.2 | Verbindung aus Bsp. 12<br>+ A[1)]<br>+ B[1)] | 0,5<br>0,5<br>0,25 | 4,01 | 13,83 |
| 9.3 | 2-(2'-Hydroxy-5'-methyl-phenyl)-benztriazol<br>+ A[1)]<br>+ B[1)] | 0,5<br>0,5<br>0,25 | 5,52 | 16,23 |
| 9.4 | 2-(2'-Hydroxy-5'-methyl-phenyl)-benztriazol<br>+ C[1)]<br>+Triethylenglykol-bis-3-(3-t.butyl-4-hydroxy-5-methylphenyl)-propionat | 0,5<br>0,5<br>0,25 | 5,44 | 17,21 |
| 9.5 | Verbindung aus Bsp. 16<br>+ A[1)]<br>+ B[1)] | 0,5<br>0,5<br>0,25 | 5,35 | 15,28 |
| 9.6 | Verbindung aus Bsp. 38<br>+ A[1)]<br>+ B[1)] | 0,5<br>0,5<br>0,25 | 5,19 | 15,40 |
| 9.7 | Verbindung aus Bsp. 12<br>+ D[2)]<br>+ B[1)] | 0,5<br>0,5<br>0,25 | 3,89 | 13,56 |
| 9.8 | Verbindung aus Bsp. 12<br>+ E[3)]<br>+ B[1)] | 0,5<br>0,5<br>0,25 | 3,45 | 12,59 |

1) A, B, C haben die in Beispiel 93 angegebene Bedeutung

2) D =

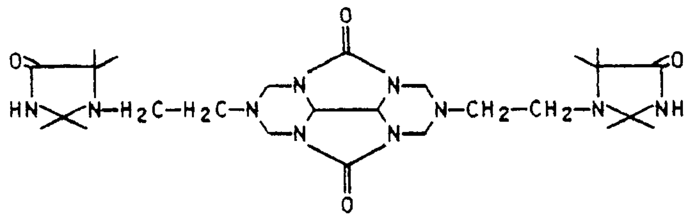

3) E =

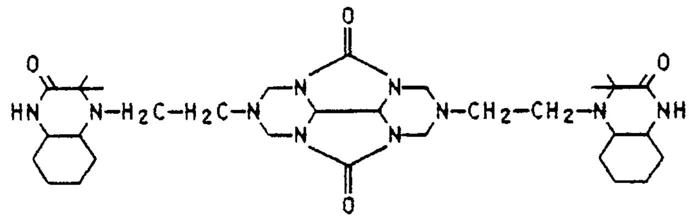

Beispiel 96

In der folgenden Meßreihe sind weitere Stabilisatormischungen aufgeführt, die in PUR-Schaumsystemen 48 Stunden im Xenotest ®450 belichtet wurden.

Tabelle 10

| | Stabilisator-Mischung | Konz. (Gew.%) | Yellowness-Index nach ASTM D 1925 im Xenotest 450 nach 0 h | 48 h |
|---|---|---|---|---|
| 10.1 | Kontrolle | - | 5,41 | 42,60 |
| 10.2 | Verbindung aus Bsp. 12<br>+ A[1)]<br>+ B[1)] | 0,5<br>0,5<br>0,25 | 6,71 | 21,49 |
| 10.3 | F[3)]<br>+ A[1)]<br>+ B[1)] | 0,5<br>0,5<br>0,25 | 6,27 | 27,46 |
| 10.4 | Verbindung aus Bsp. 12<br>+ G[4)]<br>+ B[1)] | 0,5<br>0,5<br>0,25 | 7,35 | 19,64 |
| 10.5 | Verbindung aus Bsp. 12<br>+ H[5)]<br>+ B[1)] | 0,5<br>0,5<br>0,25 | 7,60 | 22,51 |

1) A, B haben die in Beispiel 93 angegebene Bedeutung

3) F = 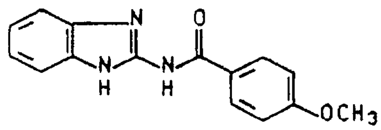 Vergleich zu US-PS 3 907 700 und US-PS 4 011 236

4) G = 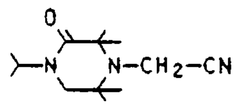

5) H= 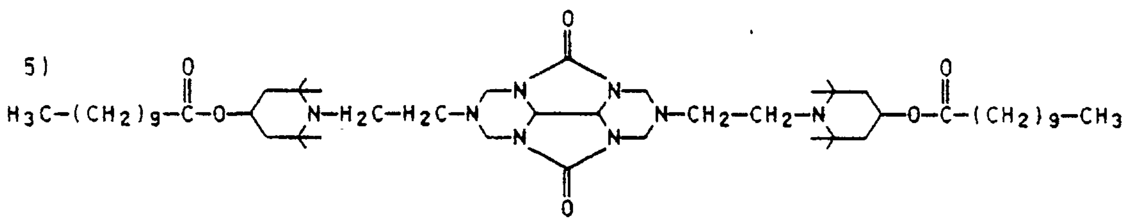

Beispiel 97

Die in Tabelle 11 aufgeführten Proben wurden analog Beispiel 93 präpariert und 96 h im Xenotest® 450 belichtet.

Tabelle 11

| | Stabilisator-Mischung | Konz. (Gew.%) | Yellowness-Index nach ASTM D 1925 im Xenotest 450 nach 0 h | 48 h | 96 h |
|---|---|---|---|---|---|
| 11.1 | Kontrolle | - | 1,4 | 24,1 | 31,7 |
| 11.2 | 2-(2'-Hydroxy-5-'methyl-phenyl)-benztriazol | 0,5 | | | |
| | + Triethylenglykol-bis-3--(3-t-butyl-4-hydroxy-5-methylphenyl-propionat | 0,5 | 2,7 | 14,7 | 23,2 |
| 11.3 | C[1)] | 0,5 | | | |
| | + Verbindung aus Bsp. 91 | 1,0 | 3,3 | 10,2 | 16,6 |
| | + Verbindung aus Bsp. 92 | 1,0 | 4,2 | 12,3 | 19,7 |

1) Beispiel 93: C

## Patentansprüche

1. Gegen Einwirkung von Licht stabilisiertes Polymer aus der Gruppe Polyolefine, Polystyrol, Styrolpolymerisate, halogenhaltige Vinylpolymere, Polyacrylate, Polymethacrylate, Polyacrylamide, Polyacrylnitril, Polyvinylalkohol und dessen Acylderivate, Polyacetate, Polyalkylenoxide, Polyphenylenoxide, Polyurethane und Polyharnstoffe, Polysulfone, Polyamide, Polyester, Polycarbonate, vernetzte Polymere aus Aldehyden und Phenolen, Harnstoff und/oder Melamin, ungesättigte Polyesterharze, Alkydharze, duroplastische und thermoplastische Acrylharze, dadurch gekennzeichnet, daß dieses als Lichtschutzmittel in Mengen von 0,01 bis 5 Gewichtsprozent, bezogen auf das zu stabilisierende Polymer, mindestens eine Verbindung der allgemeinen Formel (I)

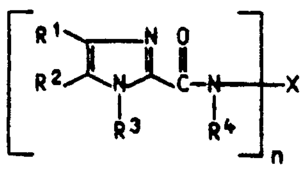

(I)

enthält, in der

n für 1 oder 2,

$R^1$ und $R^2$ oder einer der Reste $R^1$ oder $R^2$ unabhängig voneinander für Phenyl oder Heteroaryl, wobei diese Reste gegebenenfalls durch $C_1$- bis $C_{12}$-Alkyl, $C_1$- bis $C_{12}$-Alkoxy, Phenoxy, Halogen oder Trifluormethyl substituiert sind und der andere der Reste $R^1$ oder $R^2$ für Wasserstoff, $C_1$- bis $C_{12}$-Alkyl, $C_7$- bis $C_9$-Phenalkyl oder für $-COOR^5$, worin $R^5$ lineares oder verzweigtes $C_1$- bis $C_{12}$-Alkyl, $C_5$- bis $C_8$-Cycloalkyl, Phenyl oder $C_7$- bis $C_{10}$-Phen-alkyl bedeutet und wobei der Cycloalkylrest und die Phenylreste gegebenenfalls durch $C_1$- bis $C_4$-Alkyl substituiert sind, oder

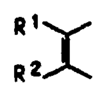

für einen ankondensierten Benzolring, der gegebenenfalls durch Hydroxy, Halogen, $C_1$- bis $C_{12}$-Alkyl, $C_1$- bis $C_{12}$-Alkoxy, $C_7$- bis $C_9$-Phenylalkyl, Phenyl, $-COOR^6$, $-CO-R^6$, $-CO-NH-R^6$, -COOH, $-O-COR^6$, $-NH-CO-R^6$, $-SO_2-R^6$ oder -CN substituiert ist, worin $R^6$ lineares oder verzweigtes $C_1$- bis $C_{12}$-Alkyl, $C_5$- bis $C_8$-Cycloalkyl, Phenyl oder $C_7$- bis $C_{10}$-Phenalkyl bedeutet und die Cycloalkyl- und Phenylreste gegebenenfalls durch $C_1$- bis $C_4$-Alkyl substituiert sind,

$R^3$ und $R^4$ für Wasserstoff oder einer der Reste $R^3$ oder $R^4$ für $C_1$- bis $C_4$-Alkyl oder $C_7$- bis $C_9$-Phenalkyl und, wenn n = 1 ist

X

a) für Phenyl, Naphthyl oder einen 5- oder 6-gliedrigen aromatischen gegebenenfalls mit einem Benzolring kondensierten Heterocyclus, wobei diese Reste gegebenenfalls durch $C_1$- bis $C_{12}$-Alkyl, $C_1$- bis $C_{12}$-Alkoxy, Methylendioxy, Ethylendioxy, Phenoxy, $C_7$- bis $C_9$-Phenylalkoxy, $C_7$- bis $C_9$-Phenylalkyl, Trifluormethyl, Hydroxy, Halogen, $-S-R^7$, $-COR^7$, $-COOR^7$, $-OCOR^7$, -COOH, -CN, $-CONH-R^7$, $-NH-R^7$, $-H(R^7)_2$ oder $-NH-COR^7$ einfach oder zweifach substituiert sind, worin $R^7$ lineares oder verzweigtes $C_1$- bis $C_{12}$-Alkyl, $C_5$- bis $C_8$-Cycloalkyl, Phenyl oder $C_7$- bis $C_{10}$-Phenalkyl, worin die Cycloalkyl-und Phenylreste gegebenenfalls durch $C_1$- bis $C_4$-Alkyl substituiert sind, oder

wenn n = 2 ist

X

b) für Phenylen oder Naphthylen, wobei diese Reste gegebenenfalls durch $C_1$- bis $C_{12}$-Alkyl, $C_1$- bis $C_{12}$-Alkoxy oder Halogen substituiert sind, oder

c) für einen Rest der Formel

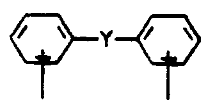

stehen, worin Y ein Rest der Formel $-(CH_2)_m$, -CO-, $-CO-(CH_2)_pCO-$, $-OCO-(CH_2)_pCOO-$, $-NH-CO-(CH_2)_p-CO-NH-$, -O-, $SO_2$-,

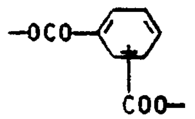 oder 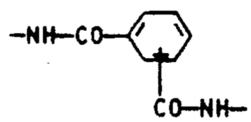 ,

m = 1, 2 oder 3 und p = 0 oder eine der Zahlen 1 bis 8 sind.

**2.** Gegen die Einwirkung von Licht stabilisiertes Polymer aus der Gruppe Polyolefine, Polystyrol, Styrolpolymerisate, halogenhaltige Vinylpolymere, Polyacrylate, Polymethacrylate, Polyacrylamide, Polyacrylnitril, Polyvinylalkohol und dessen Acylderivate, Polyacetate, Polyalkylenoxide, Polyphenylenoxide, Polyurethane und Polyharnstoffe, Polysulfone, Polyamide, Polyester, Polycarbonate, vernetzte Polymere aus Aldehyden und Phenolen, Harnstoff und/oder Melamin, ungesättigte Polyesterharze, Alkydharze, duroplastische und thermoplastische Acrylharze, dadurch gekennzeichnet, daß dieses als Lichtschutzmittel in Mengen von 0,01 bis 5 Gewichtsprozent, bezogen auf das zu stabilisierende Polymer, mindestens eine Verbindung der allgemeinen Formel (II)

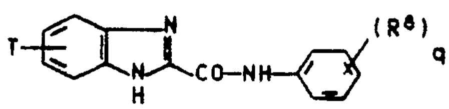 (II)

enthält, in der

- T für Wasserstoff, $C_1$- bis $C_4$-Alkyl, $C_1$- bis $C_4$-Alkoxy, Trifluormethyl, Phenyl, $C_7$- bis $C_9$-Phenalkyl oder Chlor,
- $R^8$ für $C_1$- bis $C_{12}$-Alkyl, $C_1$- bis $C_{12}$-Alkoxy, Phenoxy, $C_7$- bis $C_9$-Phenalkoxy, $C_7$- bis $C_9$-Phenalkyl, Trifluormethyl, N,N-Di-$C_1$- bis $C_6$-alkylamino, $C_2$- bis $C_{12}$-Alkanoylamino, $C_2$- bis $C_{12}$-Alkanoyloxy, Benzoyloxy, $C_7$- bis $C_9$-Phenalkanoyloxy, $C_1$- bis $C_{12}$-Alkylcarbonyl, $C_1$- bis $C_{12}$-Alkoxycarbonyl, Benzoyl, Cyano, Methylendioxy oder Ethylendioxy und
- q für 1 oder 2 stehen.

**3.** Stabilisiertes Polymer gemäß Anspruch 2, dadurch gekennzeichnet, daß

- T Wasserstoff, Methyl oder Chlor,
- $R^8$ $C_1$- bis $C_{12}$-Alkyl, $C_1$- bis $C_{12}$-Alkoxy, Methylendioxy, Ethylendioxy, Trifluormethyl oder $C_2$- bis $C_{12}$-Alkanoylamino und
- q 1 oder 2 bedeuten.

**4.** Stabilisiertes Polymer gemäß den Ansprüchen 1 bis 3, dadurch gekennzeichnet, daß das Polymer ein Polyurethan ist.

**5.** Imidazol-2-carbonsäureanilide der allgemeinen Formel (Ia)

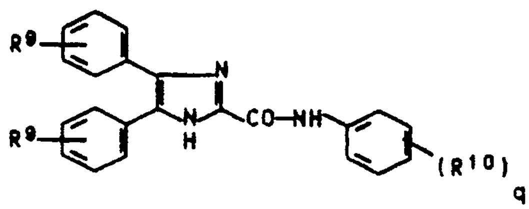

(Ia)

in der

$R^9$ Methyl, Methoxy oder Wasserstoff,

$R^{10}$ $C_1$- bis $C_{12}$-Alkyl, $C_1$- bis $C_{12}$-Alkoxy, Chlor und

q 1 oder 2 bedeuten.

6. Imidazol-2-carbonsäureanilide der allgemeinen Formel (Ib)

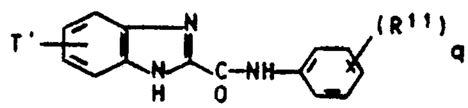

(Ib)

in der

T für Wasserstoff, q für 1 und $R^{11}$ für 4'-$CH_3$, 4'-$C_2H_5$, 2'-$C_2H_5$, 4'-i-$C_3H_7$, 4'-t-$C_4H_9$, 2'-$OC_2H_5$, 4'-$OC_2H_5$, 3'-$OC_2H_5$, 4'-$OC_6H_{13}$-n, 4'-$OC_8H_{17}$-n, 4'-$OC_{12}H_{25}$-n, 4'-$C_{10}H_{21}$-n, 4'-$C_{12}H_{25}$-n, 4 -$N(CH_3)_2$, 3'-$CF_3$, 2'-$OC_4H_9$-n, 2'-$OC_6H_{13}$-n, 2'-$OC_8H_{17}$-n, 2'-$OC_{12}H_{25}$-n, 4'-$OC_6H_5$,4'-$COC_6H_5$, 2'-$COC_6H_5$, 4'-CN, 4'-$C_4H_9$-n, 4'-NH-CO-$CH_2CH_3$, 4'-NH-CO-t-$C_4H_9$, 4'-NH-CO-$CH(C_2H_5)C_4H_9$-n, 4'-$OC_5H_{11}$-n, 4'-$OC_5H_{11}$-i, 4'-$OC_7H_{15}$-n, 4 -$OCH_2$-$CH(C_2H_5)C_4H_9$-n, 4'-$OC_9H_{19}$-n, 4'-$OC_{10}H_{21}$-n, 4'-$OCH_2$-$C_6H_5$, 4'-$OCH_2$-$CH_2$-$C_6H_5$, 4'-O-CO-$C(CH_3)_3$, 4'-O-CO-$C_2H_5$, 4'-O-CO-$CH(C_2H_5)C_4H_9$-n oder 4'-O-CO-$C_9H_{19}$ oder

T für 5- oder 6-$CH_3$, q für 1 und $R^{11}$ für 4'-t-$C_4H_9$, 4'-i-$C_3H_7$ oder4'-$CO_2C_2H_5$ oder

T für 5- oder 6-$CH_3$, q für 2 und $R^{11}$ für 2',5'-$(OC_2H_5)_2$ oder

T für 4-, 5-, 6- oder 7-$OC_2H_5$ oder für 4-, 5-, 6-, 7-$C(CH_3)_2$-$C_6H_5$, q für 1 und $R^{11}$ für 4'-$OC_2H_5$ oder

T für Wasserstoff, q für 2 und $R^{11}$ für 2', 5'$(OC_2H_5)_2$, 3',4'-O-$CH_2$-O-, 3',4'-O-$(CH_2)_2$-O-, 3',4'-$(OCH_3)_2$, 2',6'-$(CH_3)_2$ oder 2',6'-$[CH(CH_3)_2]_2$ steht.

7. Imidazol-2-carbonsäureanilide nach Anspruch 6, bei denen T für Wasserstoff, q für 1 und $R^{11}$ für 4'-$C_2H_5$, 2'-$C_2H_5$, 4'-i-$C_3H_7$, 4'-$OC_2H_5$, 3'-$OC_2H_5$, 4'-$OC_6H_{13}$-n, 4'-$OC_8H_{17}$-n, 4'-$C_{12}H_{25}$-n oder 4'-$C_4H_9$-n steht.

## Claims

1. A photostabilized polymer from the group consisting of polyolefins, polystyrene, styrene polymers, halogen-containing vinyl polymers, polyacrylates, polymethacrylates, polyacrylamides, polyacrylonitrile, polyvinyl alcohol and acyl derivatives thereof, polyacetates, polyalkylene oxides, polyphenylene oxides, polyurethanes and polyureas, polysulfones, polyamides, polyesters, polycarbonates, crosslinked polymers of aldehydes and phenols, urea or melamine, unsaturated polyester resins, alkyd resins, and thermosetting and thermoplastic acrylic resins, which contains as light stabilizer in amounts of from 0.01 to 5 percent by weight, based on the polymer to be stabilized, at least one compound of the formula (I)

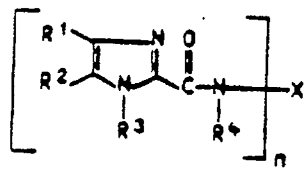

(I)

where
n is 1 or 2,
$R^1$ and $R^2$ or one of the radicals $R^1$ or $R^2$ are each independently of the other phenyl or hetaryl which are each unsubstituted or substituted by $C_1$-$C_{12}$-alkyl, $C_1$-$C_{12}$-alkoxy, phenoxy, halogen or trifluoromethyl, and the other of the radicals $R^1$ or $R^2$ is hydrogen, $C_1$-$C_{12}$-alkyl, $C_7$-$C_9$-phenalkyl or -$COOR^5$, where $R^5$ is linear or branched $C_1$-$C_{12}$-alkyl, $C_5$-$C_8$-cycloalkyl, phenyl or $C_7$-$C_{10}$-phenalkyl, where cycloalkyl and phenyl are unsubstituted or substituted by $C_1$-$C_4$-alkyl, or

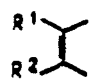

is a fused-on benzene ring which is unsubstituted or substituted by hydroxyl, halogen, $C_1$-$C_{12}$-alkyl, $C_1$-$C_{12}$-alkoxy, $C_7$-$C_9$-phenylalkyl, phenyl, -$COOR^6$, -CO-$R^6$, -CO-NH-$R^6$, -COOH, -O-$COR^6$, -NH-CO-$R^6$, -$SO_2$-$R^6$ or - CN, where $R^6$ is linear or branched $C_1$-$C_{12}$-alkyl, $C_5$-$C_8$-cycloalkyl, phenyl or $C_7$-$C_{10}$-phenalkyl and the cycloalkyl and phenyl radicals are unsubstituted or substituted by $C_1$-$C_4$-alkyl,
$R^3$ and $R^4$ are each hydrogen or one of the radicals $R^3$ or $R^4$ is $C_1$-$C_4$-alkyl or $C_7$-$C_9$-phenalkyl and,
if n is 1,
X
a) is phenyl, naphthyl or a 5- or 6-membered aromatic heterocycle which may be benzofused, these radicals being unsubstituted or monosubstituted or disubstituted by $C_1$-$C_{12}$-alkyl, $C_1$-$C_{12}$-alkoxy, methylenedioxy, ethylenedioxy, phenoxy, $C_7$-$C_9$-phenylalkoxy, $C_7$-$C_9$-phenylalkyl, trifluoromethyl, hydroxyl, halogen, -S-$R^7$, -$COR^7$, -$COOR^7$, $OCOR^7$, -COOH, -CN, -CONH-$R^7$, -NH-$R^7$, -$N(R^7)_2$ or - NH-$COR^7$, where $R^7$ is linear or branched $C_1$-$C_{12}$-alkyl, $C_5$-$C_8$-cycloalkyl, phenyl or $C_7$-$C_{10}$-phenalkyl, in which the cycloalkyl and phenyl radicals are unsubstituted or substituted by $C_1$-$C_4$-alkyl, or
if n is 2
X
b) is phenylene or naphthylene, these radicals being unsubstituted or substituted by $C_1$-$C_{12}$-alkyl, $C_1$-$C_{12}$-alkoxy or halogen, or
c) is a radical of the formula

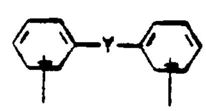

where Y is a radical of the formula $-(CH_2)_m$, -CO-, -CO-$(CH_2)_p$CO-, -OCO-$(CH_2)_p$COO-, -NH-CO-$(CH_2)_p$-CO-NH-, -O-, $SO_2$-,

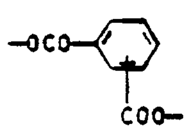

or

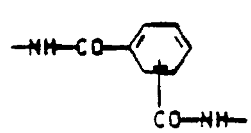

m is 1,2 or 3, and p is 0 or an integer from 7 to 8.

2. A photostabilized polymer from the group consisting of polyolefins, polystyrene, styrene polymers, halogen-containing vinyl polymers, polyacrylates, polymethacrylates, polyacrylamides, polyacrylonitrile, polyvinyl alcohol and acyl derivatives thereof, polyacetates, polyalkylene oxides, polyphenylene oxides, polyurethanes and polyureas, polysulfones, polyamides, polyesters, polycarbonates, crosslinked polymers of aldehydes and phenols, urea or melamine, unsaturated polyester resins, alkyd resins, and thermosetting and thermoplastic acrylic resins, which contains as light stabilizer in amounts of from 0.01 to 5 percent by weight, based on the polymer to be stabilized, at least one compound of the formula (II)

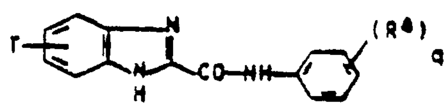

where

T is hydrogen, $C_1$-$C_4$-alkyl, $C_1$-$C_4$-alkoxy, trifluoromethyl, phenyl, $C_7$-$C_9$-phenalkyl or chlorine,

$R^8$ is $C_1$-$C_{12}$-alkyl, $C_1$-$C_{12}$-alkoxy, phenoxy, $C_7$-$C_9$-phenalkoxy, $C_7$-$C_9$-phenalkyl, trifluoromethyl, N, N-di-$C_1$-$C_6$-alkylamino, $C_2$-$C_{12}$-alkanoylamino, $C_2$-$C_{12}$-alkanoyloxy, benzoyloxy, $C_7$-$C_9$-phenalkanoyloxy, $C_1$-$C_{12}$-alkylcarbonyl, $C_1$-$C_{12}$-alkoxycarbonyl, benzoyl, cyano, methylenedioxy or ethylenedioxy, and

q is 1 or 2.

3. A stabilized polymer as claimed in claim 2, wherein

T is hydrogen, methyl or chlorine,

$R^8$ is $C_1$-$C_{12}$-alkyl, $C_1$-$C_{12}$-alkoxy, methylenedioxy, ethylenedioxy, trifluoromethyl or $C_2$-$C_{12}$-alkanoylamino and

q is 1 or 2.

4. A stabilized polymer as claimed in any of claims 1 to 3, wherein the polymer is a polyurethane.

5. An imidazole-2-carboxanilide of the formula (Ia)

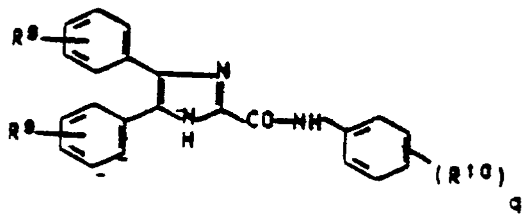

(Ia)

where

$R^9$ is methyl, methoxy or hydrogen,

$R^{10}$ is $C_1$-$C_{12}$-alkyl, $C_1$-$C_{12}$-alkoxy or chlorine,

and

q is 1 or 2.

6. An imidazole-2-carboxanilide of the formula (Ib)

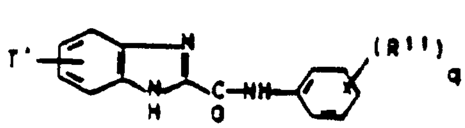

(Ib)

where
T is hydrogen, q is 1 and $R^{11}$ is 4'-$CH_3$, 4'-$C_2H_5$, 2'-$C_2H_5$, 4'-i-$C_3H_7$, 4'-t-$C_4H_9$, 2'-$OC_2H_5$, 4'-$OC_2H_5$, 3'-$OC_2H_5$, 4'-$OC_6H_{13}$-n, 4'-$OC_8H_{17}$-n, 4'-$OC_{12}H_{25}$-n, 4'-$C_{10}H_{21}$-n, 4'-$C_{12}H_{25}$-n, 4'-$N(CH_3)_2$, 3'-$CF_3$, 2'-$OC_4H_9$-n, 2'-$OC_6H_{13}$-n, 2'-$OC_8H_{17}$-n, 2'-$OC_{12}H_{25}$-n, 4'-$OC_6H_5$, 4'-$COC_6H_5$, 2'-$COC_6H_5$, 4'-CN, 4'-$C_4H_9$-n, 4'-NH-CO-$CH_2CH_3$, 4'-NH-CO-t-$C_4H_9$, 4'-NH-CO-$CH(C_2H_5)C_4H_9$-n, 4'-$OC_5H_{11}$-n, 4'-$OC_5H_{11}$-i, 4'-$OC_7H_{15}$-n, 4'-$OCH_2$-$CH(C_2H_5)C_4H_9$-n, 4-$OC_9H_{19}$-n, 4'-$OC_{10}H_{21}$-n, 4'-$OCH_2$-$C_6H_5$, 4'-$OCH_2$-$CH_2$-$C_6H_5$, 4'-O-CO-$C(CH_3)_3$, 4'-O-CO-$C_2H_5$, 4'-O-CO-$CH(C_2H_5)C_4H_9$-n or 4'-O-CO-$C_9H_{19}$ or

T is 5- or 6-$CH_3$, q is 1 and $R^{11}$ is 4'-t-$C_4H_9$, 4'-i-$C_3H_7$ or 4'-$CO_2C_2H_5$ or

T is 5- or 6-$CH_3$, q is 2 and $R^{11}$ is 2',5'-$(OC_2H_5)_2$ or

T is 4-, 5-, 6- or 7-$OC_2H_5$ or 4-, 5-, 6-, 7-$C(CH_3)_2$-$C_6H_5$, q is 1 and $R^{11}$ is 4'-$OC_2H_5$ or

T is hydrogen, q is 2 and $R^{11}$ is 2',5'-$(OC_2H_5)_2$, 3',4'-O-$CH_2$-O-, 3',4'-O-$(CH_2)_2$-O-, 3',4'-$(OCH_3)_2$, 2',6'-$(CH_3)_2$ or 2',6'-$[CH(CH_3)_2]_2$.

7. An imidazole-2-carboxanilide as claimed in claim 6, in which T is hydrogen, q is 1 and $R^{11}$ is 4'-$C_2H_5$, 2'-$C_2H_5$, 4'-i-$C_3H_7$, 4'-$OC_2H_5$, 3'-$OC_2H_5$, 4'-$OC_6H_{13-n}$, 4'-$OC_8H_{17-n}$, 4'-$C_{12}H_{25-n}$ or 4' -$C_4H_{9-n}$,.

## Revendications

1. Polymère stabilisé vis-à-vis de l'action de la lumière, qui appartient au groupe des polyoléfines, du polystyrène, des polymères du styrène, des polymères vinyliques halogénés, des polyacrylates, des polyméthacrylates, des polyacrylamides, du polyacrylonitrile, de l'alcool polyvinylique et de ses dérivés acylés, des polyacétates, des poly(oxydes d'alkylène), des poly(oxydes de phénylène), des polyuréthannes et des polyurées, des polysulfones, des polyamides, des polyesters, des polycarbonates, des polymères réticulés formés d'aldéhydes et de phénols, de l'urée et/ou de la mélamine, des résines de polyester insaturées, des résines alkyd, des résines acryliques duroplastique et thermoplastiques, caractérisé en ce qu'il contient, à titre d'agent photoprotecteur, en proportions de 0,01 à 5% en poids, par rapport au poids du polymère à stabiliser, au moins un composé répondant à la formule générale (I)

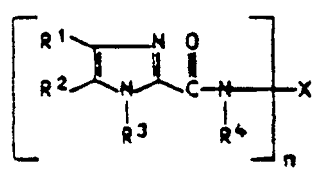

(I)

dans laquelle

n est égal à 1 ou à 2,

$R^1$ et $R^2$, ou l'un des substituants $R^1$ et $R^2$ représente, indépendamment de l'autre, des radicaux phényle ou hétéroaryle, où ces radicaux sont éventuellement substitués par des groupes alkyle en $C_1$ à $C_{12}$, alcoxy en $C_1$ à $C_{12}$, phénoxy, des atomes d'halogène ou des groupes trifluorométhyle cependant que l'autre des symboles $R^1$ et $R^2$ représente un atome d'hydrogène, un radical alkyle en $C_1$ à $C_{12}$, phénalkyle en $C_7$ à $C_9$ ou -$COOR^5$ où $R^5$ représente un radical alkyle en $C_1$ à $C_{12}$, linéaire ou ramifié, cycloalkyle en $C_5$ à $C_8$, phényle ou phénalkyle en $C_7$ à $C_{10}$ et où le groupe cycloalkyle et les groupes phényle sont éventuellement substitués par des radicaux alkyle en $C_1$ à $C_4$, ou

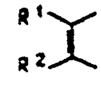

représente un noyau benzénique condensé, éventuellement substitué par des radicaux hydroxyle, des halogènes, des radicaux alkyle en $C_1$ à $C_{12}$, alcoxy en $C_1$ à $C_{12}$, phénylalkyle en $C_7$ à $C_9$, phényle, -$COOR^6$, -CO-$R^6$, -CO-NH-$R^6$, -COOH, -O-$COR^6$, -NH-CO-$R^6$, -$SO_2$-$R^6$ ou -CN, où $R^6$ représente un radical alkyle en $C_1$ à $C_{12}$, linéaire ou ramifié; cycloalkyle en $C_5$ à $C_8$, phényle ou phénalkyle en $C_7$ à $C_{10}$ et les radicaux cycloalkyle et phényle sont

éventuellement substitués par des groupes alkyle en $C_1$ à $C_4$,

$R^3$ et $R^4$ représentent des atomes d'hydrogène, ou l'un des symboles $R^3$ et $R^4$ représente un radical alkyle en $C_1$ à $C_4$ ou phénalkyle en $C_7$ à $C_9$ et lorsque n est égal à 1,

X

a) représente un radical phényle, naphtyle, ou un hétérocycle aromatique, à 5 ou 6 chaînons, éventuellement condensé avec un noyau benzénique, où ces restes ou radicaux sont éventuellement substitués une ou deux fois par des radicaux alkyle en $C_1$ à $C_{12}$, alcoxy en $C_1$ à $C_{12}$, méthylènedioxy éthylènedioxy, phénoxy, phénylalcoxy en $C_7$ à $C_9$, phénylalkyle en $C_7$ à $C_9$, trifluorométhyle, hydroxyle, des atomes d'halogènes, des radicaux $-S-R^7$, $-COR^7$, $-COOR^7$, $-OCOR^7$, -COOH, -CN, $-CONH-R^7$, $-NH-R^7$, $-N(R^7)_2$ ou $-NH-COR^7$, où $R^7$ représente un radical alkyle en $C_1$ à $C_{12}$, linéaire ou ramifié, cycloalkyle en $C_5$ à $C_8$, phényle ou phénylalkyle en $C_7$ à $C_{10}$, où les radicaux cycloalkyle et phényle sont éventuellement substitués par des radicaux alkyle en $C_1$ à $C_4$, ou

lorsque n est égal à 2,

X

b) représente un radical phénylène ou naphtylène, où ces radicaux ou restes sont éventuellement substitués par des radicaux alkyle en $C_1$ à $C_{12}$, alcoxy en $C_1$ à $C_{12}$ ou des atomes d'halogènes, ou

c) représente un radical de la formule

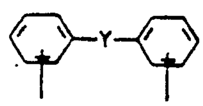

dans laquelle Y représente un groupe de la formule $-(CH_2)_m$, -CO-, $-CO-(CH_2)_pCO-$, $-OCO-(CH_2)_pCOO-$, $-NH-CO-(CH_2)_p-CO-NH-$, -O-, $SO_2$-,

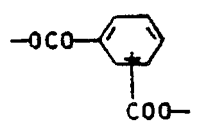

ou

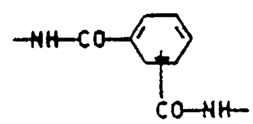

m est égal à 1, 2 ou 3 et p est égal à 0 ou représente l'un des nombres 1 à 8.

**2.** Polymère stabilisé vis-à-vis de l'action de la lumière, qui appartient au groupe des polyoléfines, du polystyrène, des polymères du styrène, des polymères vinyliques halogénés, des polyacrylates, des polyméthacrylates, des polyacrylamides, du polyacrylonitrile, de l'alcool polyvinylique et de ses dérivés acylés, des polyacétates, des poly(oxydes d'alkylène), des poly(oxydes de phénylène), des polyuréthannes et des polyurées, des polysulfones, des polyamides, des polyesters, des polycarbonates, des polymères réticulés formés d'aldéhydes et de phénols, d'urée et/ou de mélamine, des résines de polyester insaturées, des résines alkyd, des résines acryliques, duroplastiques et thermoplastiques, caractérisé en ce qu'il contient, à titre d'agent photoprotecteur, en proportions de 0,01 à 5% en poids, par rapport au polymère à stabiliser, d'au moins un composé de la formule générale (II)

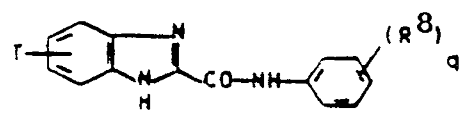

(II)

dans laquelle

R représente un atome d'hydrogène, un radical alkyle en $C_1$ à $C_4$, alcoxy en $C_1$ à $C_4$, trifluorométhyle, phényle, phénalkyle en $C_7$ à $C_9$, ou du chlore,

$R^8$ représente un radical alkyle en $C_1$ à $C_{12}$, alcoxy en $C_1$ à $C_{12}$, phénoxy, phénalcoxy en $C_7$ à $C_9$, phénalkyle en $C_7$ à $C_9$, trifluorométhyle, N,N-dialkylamino en $C_1$ à $C_8$, alcanoylamino en

$C_2$ à $C_{12}$, alcanoyloxy en $C_2$ à $C_{12}$, benzoyloxy, phénalcanoyloxy en $C_7$ à $C_9$, alkylcarbonyle en $C_1$ à $C_{12}$, alcoxycarbonyle en $C_1$ à $C_{12}$, benzoyle, cyano, méthylènedioxy ou éthylènedioxy et

q est égal à 1 ou à 2.

3. Polymère stabilisé suivant la revendication 2, caractérisé en ce que

T représente un atome d'hydrogène, le radical méthyle ou un atome de chlore,

$R^8$ représente un radical alkyle en $C_1$ à $C_{12}$, alcoxy en $C_1$ à $C_{12}$, méthylènedioxy, éthylènedioxy, trifluorométhyle, ou alcanoylamino en $C_2$ à $C_{12}$ et

q est égal à 1 ou à 2.

4. Polymère stabilisé suivant les revendications 1 à 3, caractérisé en ce que le polymère est un polyuréthanne.

5. Imidazole-2-carboxanilides de la formule générale (Ia)

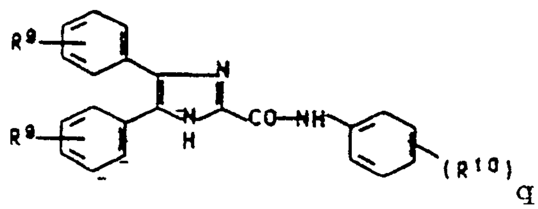

(Ia)

dans laquelle

$R^9$ représente le radical méthyle, méthoxy ou un atome d'hydrogène,

$R^{10}$ représente un radical alkyle en $C_1$ à $C_{12}$, alcoxy en $C_1$ à $C_{12}$, un atome de chlore et

q est égal à 1 ou à 2.

6. Imidazole-2-carboxanilides de la formule générale (Ib)

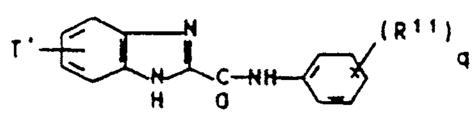

(Ib)

dans laquelle

T représente un atome d'hydrogène, q est égal à 1 et $R^{11}$ représente un radical 4'-$CH_3$, 4'-$C_2H_5$, 2'-$C_2H_5$, 4'-i-$C_3H_7$, 4'-t-$C_4H_9$, 2'-$OC_2H_5$, 4'-$OC_2H_5$, 3'-$OC_2H_5$, 4'-$OC_6H_{13}$-n, 4'-$OC_8H_{17}$-n, 4'-$OC_{12}H_{25}$-n, 4'-$C_{10}H_{21}$-n, 4'-$C_{12}H_{25}$-n, 4'-$N(CH_3)_2$, 3'-$CF_3$, 2'-$OC_4H_9$-n, 2'-$OC_6H_{13}$-n, 2'-$OC_8H_{17}$-n, 2'-$OC_{12}H_{25}$-n 4'-$OC_6H_5$, 4'-$COC_6H_5$, 2'-$COC_6H_5$, 4'-CN, 4'-$C_4H_9$-, 4'-NH-CO-$CH_2CH_3$, 4'-NH-CO-t-$C_4H_9$, 4'-NH-CO-CH-($C_2H_5$)$C_4H_9$-n, 4'-$OC_5H_{11}$-n, 4'-$OC_5H_{11}$-i, 4'-$OC_7H_{15}$-n, 4'-$OCH_2$-CH($C_2H_5$)$C_4H_9$-n, 4'-$OC_9H_{19}$-n, 4'-$OC_{10}H_{21}$-n, 4'-$OCH_2$-$C_6H_5$, 4'-$OCH_2$-$CH_2$-$C_6H_5$,4'-O-CO-C$(CH_3)_3$, 4'-O-CO-$C_2H_5$, 4'-O-CO-CH($C_2H_5$)-$C_4H_9$-nou 4'-O-CO-$C_9H_{19}$ ou bien

T représente 5- ou 6-$CH_3$, q est égal à 1 et $R^{11}$ représente 4'-t-$C_4H_9$, 4'-i-$C_3H_7$ ou 4'-$CO_2C_2H_5$ ou bien

T représente 5- ou 6-$CH_3$, q est égal à 2 et $R^{11}$ représente 2', 5'-$(OC_2H_5)_2$ ou bien

T représente 4-, 5-, 6- ou 7-$OC_2H_5$ ou représente 4-, 5-, 6-, 7-$(CH_3)_2$-$C_6H_5$, Q est égal à 1 et $R^{11}$ représente 4'-$OC_2H_5$ au bien

T représente H, q est égal à 2 et $R^{11}$ représente 2', 5'-$(OC_2H_5)_2$, 3', 4'-O-$CH_2$-O-, 3', 4'-O-$(CH_2)_2$-O-, 3', 4'-$(OCH_3)_2$, 2', 6'-$(CH_3)_2$ ou 2', 6'-$[CH(CH_3)_2]_2$.

7. Imidazole-2-carboxanilides selon la revendication 6, caractérisés en ce que T représente un atome d'hydrogène, q est égal à 1 et $R^{11}$ représente 4'-$C_2H_5$, 2'-$C_2H_5$, 4'-i-$C_3H_7$, 4'-$OC_2H_5$, 3'-$OC_2H_5$, 4'-

$OC_6H_{13}$, 4'-$OC_8H_{17}$-n, 4'-$C_{12}H_{25}$ ou 4'-$C_4H_9$-n.